(19) Europäisches Patentamt
European Patent Office
Office européen des brevets

(11) Veröffentlichungsnummer: **0 402 688 B1**

(12) # EUROPÄISCHE PATENTSCHRIFT

(45) Veröffentlichungstag der Patentschrift: **16.02.94**

(51) Int. Cl.⁵: **C07C 205/37**, C07C 201/12, C07C 215/80, C07C 213/00

(21) Anmeldenummer: **90110026.3**

(22) Anmeldetag: **26.05.90**

(54) **1,3-Di-arylmethoxy-4,6-dinitrobenzole, Verfahren zu ihrer Herstellung und Verfahren zur Herstellung von 4,6-Diaminoresorcin.**

(30) Priorität: **10.06.89 DE 3919045**

(43) Veröffentlichungstag der Anmeldung:
**19.12.90 Patentblatt 90/51**

(45) Bekanntmachung des Hinweises auf die Patenterteilung:
**16.02.94 Patentblatt 94/07**

(84) Benannte Vertragsstaaten:
**BE CH DE FR GB IT LI NL**

(56) Entgegenhaltungen:
**EP-A- 0 312 931**
**US-A- 4 745 232**

(73) Patentinhaber: **BAYER AG**

**D-51368 Leverkusen(DE)**

(72) Erfinder: **Blank, Heinz Ulrich, Dr.**
**Am Geusfelde 35**
**D-5068 Odenthal(DE)**
Erfinder: **Heinz, Uwe, Dr.**
**Silesiusstrasse 78**
**D-5000 Köln 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 402 688 B1

## Beschreibung

Die vorliegende Erfindung betrifft neue Di-arylmethoxydinitro-benzole, ein Verfahren zu ihrer Herstellung aus Dihalogen-dinitro-benzolen und Arylmethanolen, sowie deren weitere Umwandlung durch katalytische Hydrierung zu 4,6-Diaminoresorcin.

Diaminoresorcin wird als Monomerbaustein zur Synthese von Polybenzoxazolen eingesetzt, die beispielsweise als Ausgangsmaterial für elektroaktive Polymere dienen (DE-OS 33 41 627).

Derzeit wird Diaminoresorcin durch Nitrierung von Diacetylresorcin, Abspalten der Schutzgruppen und Reduktion der Nitrogruppen an einem Pd/A-Kohle-Katalysator dargestellt (Macromolecules 14 (1981), 909). Der kritische Schritt hierbei ist die Nitrierung. In Salpetersäure gelingt sie nur mit etwa 30 %iger Ausbeute. Da außerdem in hoher Verdünnung gearbeitet werden muß, beträgt die Raumausbeute nur etwa 10 g/l bzw. 0,05 Mol/l. Die Abspaltung der Schutzgruppe in einem weiteren Verfahrensschritt erfolgt mit ähnlich ungünstigen Raumausbeuten. Als Nebenprodukt wird Styphninsäure (2,4,6-Trinitroresorcin) gebildet, die bei erhöhter Temperatur zu Verpuffungen führt (Beilstein, Band 6 Hauptwerk, S. 830) und daher ein Sicherheitsproblem darstellt. Da die Reaktion einerseits stark exotherm ist und andererseits exotherme Zersetzungsreaktionen bei einer Temperatur eintreten, die nur wenig oberhalb der Raumtemperatur liegt, ist eine sichere Reaktionsführung schwierig.

Nach US 4.745.232 soll die Bildung von Dinitroresorcin aus Diacetylresorcin ohne die unerwünschte Bildung von Styphninsäure mit 65 %iger Ausbeute gelingen, wenn mit "weißer" Salpetersäure (gemeint ist offenbar eine $HNO_3$, die frei ist von $NO_2$) und unter Zusatz von Harnstoff als Reagens zur Kontrolle der Nitrosylionen gearbeitet wird bzw. wenn mit Salpetersäure in Schwefelsäure gearbeitet wird. Die Raumausbeute verbessert sich hierbei auf 65 g/l (0,35 Mol/l) bzw. auf 40 g/l (0,2 Mol/l), jedoch ist für diese Verbesserung der genannte erhöhte Aufwand erforderlich.

Wegen der genannten Schwierigkeiten ist versucht worden, Diaminoresorcin von einem anderen Ausgangsmaterial her zu gewinnen. So wird in EP 266 222 vorgeschlagen, 1,2,3-Trichlorbenzol in einem Schwefelsäure/Salpetersäure-Gemisch zu 1,2,3-Trichlor-4,6-dinitro-benzol zu nitrieren und diese Verbindung mit einem Alkalihydroxid in Methanol/Wasser in das 1,3-Dihydroxy-2-chlor-4,6-dinitrobenzol zu überführen. Dessen Reduktion am Pd/A-Kohle-Katalysator führt zu Diaminoresorcin. Dieses Verfahren leidet jedoch an dem Nachteil, daß die Dihydroxyverbindung der zweiten Verfahrensstufe mit Ethylacetat extrahiert werden muß, wodurch die Raumausbeute auf etwa 75 g/l (0,32 Mol/l) sinkt; nach der genannten Extraktion wird die Dihydroxyverbindung durch Eindampfen der Ethylacetatphase isoliert.

Die vorliegende Erfindung betrifft 1,3-Di-arylmethoxy-4,6-dinitro-benzole der Formel

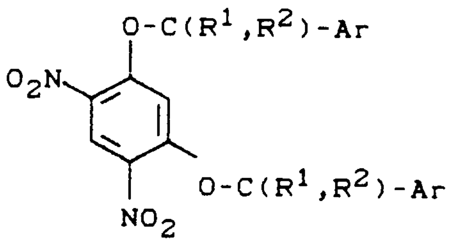

(I),

in der

$R^1$ und $R^2$ unabhängig voneinander Wasserstoff oder $C_1$-$C_4$-Alkyl bedeuten und

Ar für $C_6$-$C_{12}$-Aryl steht.

Die Erfindung betrifft weiterhin ein Verfahren zur Herstellung von 1,3-Di-arylmethoxy-4,6-dinitro-benzolen der Formel (I), das dadurch gekennzeichnet ist, daß man 1,3-Dihalogen-4,6-dinitro-benzole der Formel

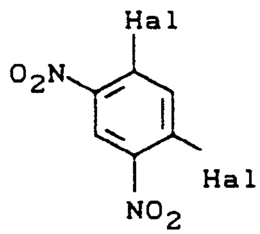

(III),

in der

Hal für Chlor oder Brom steht,

mit 2 bis 5 Mol eines Arylmethanols der Formel

$Ar-C(R^1,R^2)OH$ (IV),

in der

$R^1$, $R^2$ und Ar die genannte Bedeutung haben,

und 2 bis 5 Äquivalenten einer starken Base im Temperaturbereich von 0 bis 100 °C in Gegenwart eines inerten Lösungsmittels umsetzt.

Die Erfindung betrifft weiterhin ein Verfahren zur Herstellung von 4,6-Diaminoresorcin, das dadurch gekennzeichnet ist, daß man

a) ein 1,3-Dihalogen-4,6-dinitro-benzol der Formel (III) mit 2 bis 5 Mol eines Arylmethanols der Formel (IV), wobei Hal, $R^1$, $R^2$ und Ar den genannten Bedeutungsumfang haben, und 2 bis 5 Äquivalenten einer starken Base im Temperaturbereich von 0 bis 100 °C in flüssiger Phase umsetzt und

b) das gemäß a) erhaltene 1,3-Di-arylmethoxy-4,6-dinitro-benzol der Formel (I) in heterogener Phase unter Verwendung eines Platinmetall-Träger-Katalysators in einer Menge von 0,1 bis 10 Gew.-% des Platinmetalls, bezogen auf das Gewicht des 1,3-Di-arylmethoxy-4,6-dinitro-benzols, bei 1 bis 30 bar $H_2$-Druck und im Temperaturbereich von 20 bis 100 °C hydriert.

$R^1$ und $R^2$ bedeuten unabhängig voneinander Wasserstoff oder $C_1$-$C_4$-Alkyl, wie Methyl, Ethyl, Propyl, Isopropyl, Butyl, Isobutyl oder tert.-Butyl, in bevorzugter Weise Wasserstoff oder Methyl, in besonders bevorzugter Weise Wasserstoff.

Ar steht für $C_6$-$C_{12}$-Aryl, wie Phenyl, Naphthyl oder Bi-phenylyl, bevorzugt für Phenyl.

Hal steht für Chlor oder Brom, bevorzugt für Chlor.

Die Erfindung betrifft bevorzugterweise 1,3-Di-phenylmethoxy-4,6-dinitro-benzole der Formel

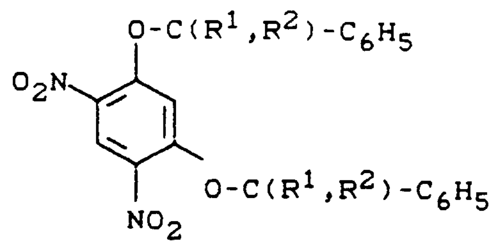

(II),

in der

$R^1$ und $R^2$ unabhängig voneinander Wasserstoff oder $C_1$-$C_4$-Alkyl bedeuten.

In besonders bevorzugter Weise betrifft die Erfindung das 1,3-Dibenzyloxy-4,6-dinitro-benzol.

Das Herstellungsverfahren für die Stoffe der Formel (I) geht von 1,3-Dihalogen-4,6-dinitro-benzolen der Formel (III) aus, welche durch Nitrieren von m-Dichlorbenzol oder m-Dibrombenzol hergestellt werden kann. Diese Ausgangsprodukte sind in einer Ausbeute von 70 % und einer Raumausbeute von 0,47 Mol/l zugänglich. Bei dieser Nitrierung ist auch eine höhere Temperatur als bei der Nitrierung von Diacetylresorcin zulässig, ohne daß unerwünschte Zersetzungsreaktionen eintreten (DE-OS 34 08 301).

Das Ausgangsmaterial (III) wird mit 2 bis 5 Mol, bevorzugt mit 2 bis 3 Mol, eines Arylmethanols der Formel (IV) und mit 2 bis 5 Äquivalenten, bevorzugt mit 2 bis 3 Äquivalenten, einer starken Base umgesetzt. Als starke Base werden eine oder mehrere aus der Gruppe der Alkalimetalle, wie Lithium,

Natrium, Kalium, Rubidium oder Cäsium, bevorzugt Natrium oder Kalium, der Erdalkalimetalle, wie Magnesium, Calcium, Strontium oder Barium, bevorzugt Magnesium oder Calcium, der Alkalimetallhydroxide, wie Lithiumhydroxid, Natriumhydroxid, Kaliumhydroxid, Rubidiumhydroxid, Cäsiumhydroxid, bevorzugt Natriumhydroxid oder Kaliumhydroxid, der Erdalkalimetallhydroxide, wie Magnesiumhydroxid, Calciumhydroxid, Strontiumhydroxid, Bariumhydroxid, bevorzugt Magnesiumhydroxid oder Calciumhydroxid, der Alkalimetallcarbonate, wie Lithiumcarbonat, Natriumcarbonat, Kaliumcarbonat, Rubidiumcarbonat, Cäsiumcarbonat, bevorzugt Natriumcarbonat oder Kaliumcarbonat, und der Erdalkalimetallcarbonate, wie Magnesiumcarbonat, Calciumcarbonat, Strontiumcarbonat oder Bariumcarbonat, bevorzugt Magnesiumcarbonat oder Calciumcarbonat, eingesetzt. In bevorzugter Weise wird eine starke Base aus der Gruppe der Alkalimetalle, der Alkalimetallhydroxide und Alkalimetallcarbonate, in besonders bevorzugter Weise aus der Gruppe Natriummetall, Natriumhydroxid und Natriumcarbonat, eingesetzt. Ebenso können die Arylmethanole auch direkt in Form ihrer Alkoholate eingesetzt werden.

Die Reaktion wird im Temperaturbereich von 0 bis 100 °C, bevorzugt im Bereich von 40 bis 70 °C, durchgeführt.

Als inertes Lösungsmittel kommt ein aliphatischer, aromatischer oder alkylaromatischer Kohlenwasserstoff, ein halogenierter aromatischer Kohlenwasserstoff oder zusätzliches Arylmethanol der Formel (IV) über die genannten 2 bis 5 Mol hinaus in Frage, sofern ein solches Arylmethanol bei der gewünschten Reaktionstemperatur flüssig ist. Beispiele für solche Lösungsmittel sind: Pentan, Hexan, Heptan, Octan, Decan, Dodecan und höhere geradkettige oder verzweigte aliphatische Kohlenwasserstoffe, sowie Gemische daraus, wie Ligroin oder Petrolether, Benzol, Toluol, Ethylbenzol, Chlorbenzol, Brombenzol, Dichlorbenzol, Dibrombenzol, Chlortoluol, Dichlortoluol oder Cyclohexan. Selbstverständlich können auch Gemische mehrerer der genannten Lösungsmittel eingesetzt werden.

Beispiele für Arylmethanole, die im Überschuß über die genannten 2 bis 5 Äquivalente eingesetzt werden können und damit gleichzeitig als Reaktionspartner und inertes Lösungsmittel dienen, sind Benzylalkohol und $\alpha$-Phenylethanol. In bevorzugter Weise sei hierbei ein solcher Einsatz im Falle des Benzylalkohols genannt. Hierbei wird ein solches Arylmethanol, bevorzugt der Benzylalkohol, in einer Menge von 3 bis 20 Gew.-Teilen, bevorzugt 3 bis 10 Gew.-Teilen, bezogen auf 1 Gew.-Teil des Dihalogen-dinitro-benzols, eingesetzt.

Die Reaktion ist nicht abhängig vom Druck über dem Reaktionsgemisch und wird daher der Einfachheit halber bei Normaldruck durchgeführt. Ein erhöhter Druck könnte dann sinnvoll sein, wenn ein niedrigsiedendes Lösungsmittel in der flüssigen Phase gehalten werden soll; in diesem Falle wird bevorzugt unter dem sich automatisch einstellenden Eigendruck gearbeitet.

Die erfindungsgemäße Umsetzung der Dihalogen-dinitro-benzole (III) mit den Arylmethanolen (IV) in Gegenwart der genannten Base verläuft schnell (Laboransätze im allgemeinen innerhalb von 30 Min.) und ergibt hohe Ausbeuten, die in der Regel 90 % überschreiten, Das Reaktionsprodukt ist in vielen Fällen im Reaktionsmedium unlöslich und kann daher durch einfaches Filtrieren isoliert werden, Anschließend können die anorganischen Nebenprodukte mit Wasser entfernt werden, wozu das Reaktionsprodukt (I) in Wasser angeschlämmt und erneut gesaugt wird, Es ist jedoch auch möglich, das ursprünglich abfiltrierte Reaktionsprodukt auf der Nutsche mit Wasser zu waschen. Bei großen Ansätzen kann selbstverständlich anstelle der Filtration das Zentrifugieren oder andere dem Fachmann bekannte Maßnahmen treten. Die erzielbare Raumausbeute beträgt etwa 0,75 Mol/l und ist daher wesentlich höher als bei den bisher bekannten Verfahren.

Die Di-arylmethoxy-dinitro-benzole (I) können durch katalytische Hydrierung in Diaminoresorcin übergeführt werden. Hierbei erfolgt eine gleichzeitige Hydrierung der Nitrogruppen zu den Aminogruppen und eine Abspaltung der Arylmethoxy-Gruppen. Als Katalysatoren werden Platinmetalle, wie Palladium, Platin, Rhodium oder Ruthenium, bevorzugt Palladium oder Platin, auf einem geeigneten Träger, eingesetzt. Geeignete Träger, wie A-Kohle, $SiO_2$, $Al_2O_3$, Bimsstein und andere sind dem Fachmann bekannt, Der Platinmetall-Träger-Katalysator wird in einer Menge von 0,1 bis 10 Gew.-% des Platinmetalls, bevorzugt 0,2 bis 2 Gew.-%, bezogen auf das Gewicht von (I), eingesetzt. Die Hydrierung wird bei einem $H_2$-Druck von 1 bis 30 bar und im Temperaturbereich von 20 bis 100 °C, bevorzugt im Bereich von 20 bis 50 °C, durchgeführt.

Als flüssiges Reaktionsmedium für die Hydrierung kann ein Alkohol, ein Ether, ein aromatischer oder alkylaromatischer Kohlenwasserstoff, eine organische Säure oder ein Gemisch mehrerer von ihnen eingesetzt werden. Beispiele für solche flüssigen Reaktionsmedien sind: Methanol, Ethanol, Propanol, Isopropanol, Butanol, Isobutanol, Tetrahydrofuran, Dioxan, Benzol, Toluol, Xylol, Dimethoxy-ethan, Diethoxy-ethan, 1,2-Dimethoxy-propan und andere Glykolmono- und -diether, Essigsäure, Propionsäure oder Gemische von ihnen. In bevorzugter Weise eignen sich als flüssige Reaktionsmedien für die Hydrierung Methanol, Ethanol, Propanol, Butanol, Tetrahydrofuran, Dioxan, Dimethoxy-ethan, Diethoxy-ethan, 1,2-Dimethoxy-propan und andere Glykolmono- und -diether, Essigsäure, Propionsäure, Toluol oder ein Gemisch mehrerer von ihnen.

Im Falle wasserlöslicher flüssiger Reaktionsmedien kann der organische Anteil dieses Reaktionsmediums bis zu 75 Gew.-% durch Wasser oder verdünnte wäßrige Mineralsäure ersetzt werden. Das gesamte flüssige Reaktionsmedium für die Hydrierung wird in einer Menge von 5 bis 25 Gew.-Teilen, bevorzugt 7 bis 15 Gew.-Teilen, bezogen auf 1 Gew.-Teil (1), eingesetzt.

Es ist bekannt, daß bei katalytischen Hydrierungen von Nitroverbindungen zu den zugehörigen Aminen für eine ausreichende Löslichkeit des Ausgangsmaterials im Reaktionsmedium zu sorgen ist; des weiteren ist bekannt, daß die entstehenden Amine, wie Amine allgemein, Platinmetall-Katalysatoren im Verlaufe der Reaktion inhibieren, so daß ein Zusatz von Säure zur Bindung der entstehenden Amine günstig ist (Houben-Weyl, Handbuch der organischen Chemie, Band 4/1c, S. 509 (1980)). Im vorliegenden Fall ist es daher überraschend, daß die katalytische Hydrierung beispielsweise in einem Reaktionsmedium aus Tetrahydrofuran und einer wäßrigen Mineralsäure mit hohen Ausbeuten gelingt, obwohl die Nitroverbindung in diesem Reaktionsmedium nahezu unlöslich ist, Ebenso ist es überraschend, daß die Reduktion in einem Alkohol auch ohne den Zusatz von Säure gelingt, obwohl das entstehende Produkt sogar eine Diaminoverbindung ist.

## Beispiel 1

1,3-Dibenzyloxy-4,6-dinitro-benzol, NaOH als Base

Zu 130 ml Benzylalkohol wurden unter Rühren 9,60 g (0,24 Mol) Natriumhydroxid gegeben. Nach vollständiger Lösung wurden 23,7 g (0,1 Mol) 1,3-Dichlor-4,6-dinitro-benzol zugegeben. Dabei trat eine exotherme Reaktion ein, Das Edukt wurde so zugegeben, daß eine Temperatur von 50 °C nicht überschritten wurde. Nach beendeter Zugabe wurde 30 Min. bei 50 °C nachgerührt. Es wurde auf Raumtemperatur abgekühlt und abgesaugt. Das Nutschgut wurde zur Entfernung von anorganischen Salzen in 500 ml Wasser aufgeschlämmt und wieder abgesaugt und getrocknet. Es wurden 34,23 g (0,09 Mol = 88,9 %) Produkt erhalten. Die Substanz ist nach Dünnschichtchromatographie (Eluationsmittel 50 Teile Xylol, 20 Teile Ethylacetat, 10 Teile Essigsäure) einheitlich.

| Elementaranalyse: | ber. | gef. |
|---|---|---|
| C | 63,4 % | 62,9 % |
| H | 4,2 % | 4,4 % |
| N | 7,4 % | 7,4 % |

IR-, $^{1}$H-NMR- und Massenspektren stehen mit der Struktur in Einklang.

## Beispiel 2

1,3-Dibenzyloxy-4,6-dinitro-benzol, Natrium als Base

11 g (0,48 Mol) Natrium wurden in 400 ml Benzylalkohol aufgelöst. Anschließend wurde auf Raumtemperatur gekühlt und das 1,3-Dichlor-4,6-dinitro-benzol (49,3 g = 0,208 Mol), so zugesetzt, daß die Temperatur 55 °C nicht überstieg. Dabei wurde mit einem Eisbad gekühlt. Es wurde 15 Min. bei 50 °C nachgerührt, dann auf 15 °C abgekühlt und das Produkt abgesaugt. Das Nutschgut wurde in 1 l Wasser angeschlämmt, 1 Stunde gerührt und wieder abgesaugt. Nach dem Waschen mit Wasser und dem Trocknen verblieben 70,4 g (0,19 Mol = 91,4 %) Produkt.

## Beispiel 3

4,6-Diaminoresorcin-dihydrochlorid, Methanol als Lösungsmittel

57 g (0,15 Mol) 1,3-Dibenzyloxy-4,6-dinitro-benzol wurden in 700 ml Methanol gegeben. Es wurden 5 g Pd/C (5 %) zugesetzt. Es wurde bei 75 °C und 3.5 bar Wasserstoffdruck solange hydriert, bis kein Wasserstoff mehr aufgenommen wurde. Der Ansatz wurde mit 500 ml Salzsäure (ca. 37 %ig) versetzt und abgesaugt. Das Nutschgut wurde 5 mal mit je 100 ml Wasser gewaschen. Die vereinigten Waschwässer wurden wieder mit 500 ml Salzsäure (ca. 37 %ig) versetzt und 1 Stunde gerührt. Der Niederschlag wurde abgesaugt, zur Verdrängung von Wasser 4 mal mit 100 ml Aceton gewaschen und im Exsikkator getrocknet. Man erhielt 26,34 g (0,124 Mol = 82,4 %) Produkt. Alle Arbeiten wurden unter Stickstoff

durchgeführt.

| Elementaranalyse | ber. | gef. |
|---|---|---|
| C | 33,8 % | 33,5 % |
| H | 4,7 % | 4,5 % |
| N | 13,1 % | 12,9 % |
| Cl | 33,3 % | 32,9 %. |

Beispiel 4

4,6-Diaminoresorcin-dihydrochlorid, Tetrahydrofuran/Salzsäure als Lösungsmittel

57 g (0,15 Mol) 1,3-Dibenzyloxy-4,6-dinitro-benzol wurden in 500 ml Salzsäure (ca. 37 %ig) und 200 ml Tetrahydrofuran gegeben. Es wurden 20 g Pd/C (5 %) zugesetzt. Es wurde bei 75 °C und 3,5 bar Wasserstoffdruck solange hydriert, bis kein Wasserstoff mehr aufgenommen wurde. Der Ansatz wurde abgesaugt. Das Nutschgut wurde 3 mal mit je 250 ml Wasser gewaschen. Die vereinigten Waschwässer wurden wieder mit 1.000 ml Salzsäure (ca. 37 %ig) versetzt und 1 Stunde gerührt. Der Niederschlag wurde abgesaugt, zur Verdrängung von Wasser 4 mal mit 100 ml Aceton gewaschen und im Exsikkator getrocknet. Man erhielt 25,00 g (0,117 Mol = 78,2 %) Produkt. Alle Arbeiten wurden unter Stickstoff durchgeführt.

Beispiele 5-10 (Reduktion)

Die Beispiele 5 bis 10 wurden in gleicher Art wie Beispiel 3 durchgeführt. Die verwendeten Lösungsmittel sowie die Einsatzmengen und Ausbeuten sind in Tabelle 1 zusammengefaßt.

Tabelle 1

| Bsp. | Einsatz (g) 1,3-Dibenzyl-oxy-4,6-dinitrobenzol | Lösungsmittel Art | Lösungsmittel Menge ml | Katalysator 5 % Pd/C g | Temperatur °C | $H_2$-Druck bar | Ausbeute g | Ausbeute % d.Th. |
|---|---|---|---|---|---|---|---|---|
| 5 | 50 | Methanol | 300 | 2,1 | 50 | 3,5 | 26,2 | 93,7 |
| 6 | 24,5 | Methanol | 300 | 0,5 | 50 | 3,5 | 12,2 | 89 |
| 7 | 24,5 | 1-Butanol | 300 | 2,1 | 50 | 3,5 | 11,7 | 84,9 |
| 8 | 24,5 | 1,2-Dimethoxyethan | 300 | 2,1 | 50 | 3,5 | 11,7 | 85,5 |
| 9 | 24,5 | Essigsäure/Wasser = 4/1 | 300 | 2,1 | 50 | 3,5 | 10,8 | 78,5 |
| 10 | 24,5 | Toluol | 300 | 2,1 | 50 | 3,5 | 11,2 | 81,4 |

**Patentansprüche**

**1.** 1,3-Di-arylmethoxy-4,6-dinitro-benzole der Formel

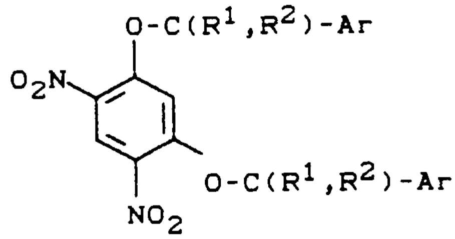

,

in der

R[1] und R[2] unabhängig voneinander Wasserstoff oder $C_1$-$C_4$-Alkyl bedeuten und

Ar für $C_6$-$C_{12}$-Aryl steht.

**2.** 1,3-Di-phenylmethoxy-4,6-dinitro-benzole der Formel

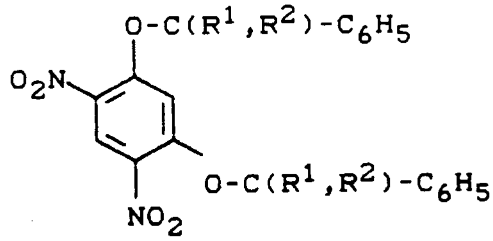

,

in der

R[1] und R[2] unabhängig voneinander Wasserstoff oder $C_1$-$C_4$-Alkyl bedeuten.

**3.** 1,3-Dibenzyloxy-4,6-dinitro-benzol.

**4.** Verfahren zur Herstellung von 1,3-Di-arylmethoxy-4,6-dinitro-benzolen der Formel

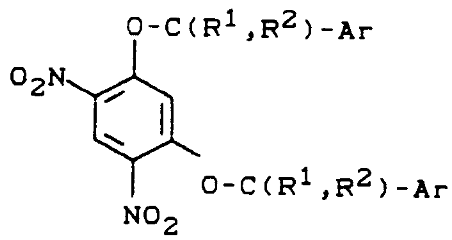

,

in der

R[1] und R[2] unabhängig voneinander Wasserstoff oder $C_1$-$C_4$-Alkyl bedeuten und

Ar für $C_6$-$C_{12}$-Aryl steht,

dadurch gekennzeichnet, daß man 1,3-Dihalogen-4,6-dinitro-benzole der Formel

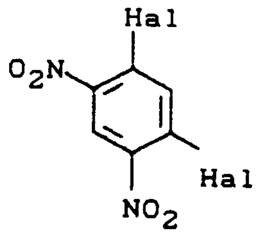
,

in der

Hal für Chlor oder Brom steht,

mit 2 bis 5 Mol, bevorzugt 2 bis 3 Mol, eines Arylmethanols der Formel

$Ar-C(R^1,R^2)OH$ ,

in der

$R^1$, $R^2$ und Ar die genannte Bedeutung haben,

und mit 2 bis 5 Äquivalenten, bevorzugt mit 2 bis 3 Äquivalenten, einer starken Base im Temperaturbereich von 0 bis 100 °C, bevorzugt 40 bis 70 °C, in Gegenwart eines inerten Lösungsmittels umsetzt.

5. Verfahren nach Anspruch 4, dadurch gekennzeichnet, daß man als starke Base eine oder mehrere aus der Gruppe der Alkalimetalle, Erdalkalimetalle, Alkalimetallhydroxide, Erdalkalimetallhydroxide, Alkalimetallcarbonate und Erdalkalimetallcarbonate, bevorzugt aus der Gruppe Alkalimetalle, Alkalimetallhydroxide und Alkalimetallcarbonate, besonders bevorzugt aus der Gruppe Natriummetall, Natriumhydroxid und Natriumcarbonat, einsetzt.

6. Verfahren nach Anspruch 4, dadurch gekennzeichnet, daß als inertes Lösungsmittel ein aliphatischer, aromatischer oder alkylaromatischer Kohlenwasserstoff, ein halogenierter aromatischer Kohlenwasserstoff oder zusätzliches Arylmethanol, sofern dies bei der Reaktionstemperatur flüssig ist, in einer Menge von 3 bis 20 Gew.-Teilen, bevorzugt 3 bis 10 Gew.-Teilen, bezogen auf das Gewicht des Dihalogen-dinitro-benzols, eingesetzt wird.

7. Verfahren nach Anspruch 5, dadurch gekennzeichnet, daß Benzylalkohol oder α-Phenyl-ethanol als Reaktionspartner und als Lösungsmittel in einer Menge von 3 bis 20 Gew.-Teilen, bevorzugt 3 bis 10 Gew.-Teilen, bezogen auf 1 Gew.-Teil Dihalogen-dinitro-benzol, eingesetzt wird.

8. Verfahren zur Herstellung von 4,6-Diaminoresorcin, dadurch gekennzeichnet, daß man

a) ein 1,3-Dihalogen-4,6-dinitro-benzol der Formel

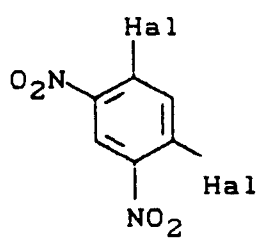
,

in der

Hal für Chlor oder Brom steht,

mit 2 bis 5 Mol, bevorzugt mit 2 bis 3 Mol, eines Arylmethanols der Formel

$Ar-C(R^1,R^2)OH$ ,

in der

$R^1$, $R^2$ unabhängig voneinander Wasserstoff oder $C_1$-$C_4$-Alkyl bedeuten und

Ar für $C_6$-$C_{12}$-Aryl steht,

und 2 bis 5 Äquivalente, bevorzugt 2 bis 3 Äquivalente einer starken Base im Temperaturbereich von 0 bis 100 °C, bevorzugt 40 bis 70 °C, in flüssiger Phase umsetzt und

b) das gemäß a) erhaltene 1,3-Di-arylmethoxy-4,6-dinitro-benzol der Formel

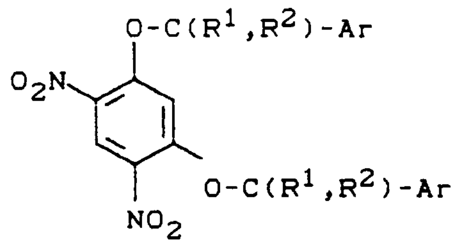

,

in der

$R^1$, $R^2$ und Ar die genannte Bedeutung haben,

in heterogener Phase unter Verwendung eines Platinmetall-Träger-Katalysators in einer Menge von 0,1 bis 10 Gew.-%, bevorzugt 0,2 bis 2 Gew.-%, bezogen auf das Gewicht des 1,3-Di-arylmethoxy-4,6-dinitro-benzols, bei 1 bis 30 bar $H_2$-Druck und im Temperaturbereich von 20 bis 100 °C, bevorzugt 20 bis 50 °C, hydriert.

**9.** Verfahren nach Anspruch 8, dadurch gekennzeichnet, daß als flüssiges Reaktionsmedium für die Stufe b) ein Alkohol, ein Ether, ein aromatischer oder alkylaromatischer Kohlenwasserstoff oder ein Gemisch mehrerer von ihnen in einer Menge von 5 bis 25 Gew.-Teilen, bevorzugt 7 bis 15 Gew.-Teilen, bezogen auf 1 Gew.-Teil Di-arylmethoxy-dinitro-benzol, eingesetzt wird.

**10.** Verfahren nach Anspruch 9, dadurch gekennzeichnet, daß als flüssiges Reaktionsmedium für die Stufe b) Methanol, Ethanol, Propanol, Butanol, Tetrahydrofuran, Dioxan, Toluol, Dimethoxy-ethan, Diethoxy-ethan, 1,2-Dimethoxy-propan und andere Glykolmono- und -diether, Essigsäure, Propionsäure oder ein Gemisch mehrerer von ihnen eingesetzt wird, wobei im Falle wasserlöslicher Reaktionsmedien bis zu 75 Gew.-% dieses Reaktionsmediums durch Wasser oder verdünnte wäßrige Mineralsäure ersetzt sein kann.

**Claims**

**1.** 1,3-Di-arylmethoxy-4,6-dinitro-benzenes of the formula

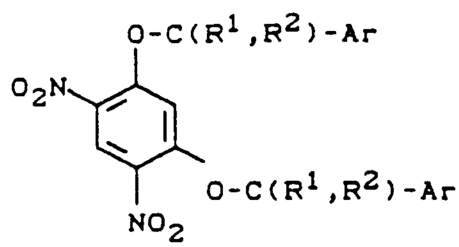

,

in which

$R^1$ and $R^2$ independently of one another denote hydrogen or $C_1$-$C_4$-alkyl and

Ar represents $C_6$-$C_{12}$-aryl.

**2.** 1,3-Di-phenylmethoxy-4,6-dinitro-benzenes of the formula

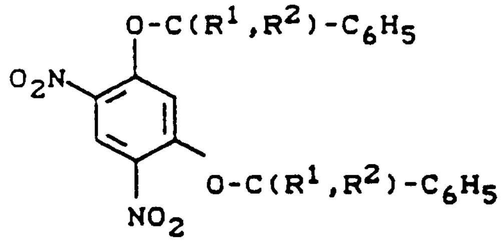

,

in which

$R^1$ and $R^2$ independently of one another denote hydrogen or $C_1$-$C_4$-alkyl.

**3.** 1,3-Dibenzyloxy-4,6-dinitro-benzene.

**4.** Process for the preparation of 1,3-di-arylmethoxy-4,6-dinitro-benzenes of the formula

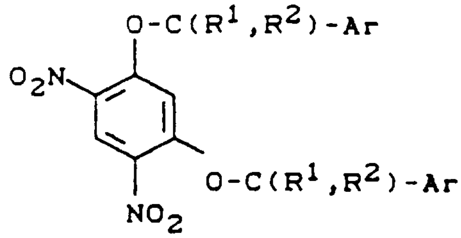

,

in which

$R^1$ and $R^2$ independently of one another denote hydrogen or $C_1$-$C_4$-alkyl and

Ar represents $C_6$-$C_{12}$-aryl,

characterized in that 1,3-dihalogeno-4,6-dinitro-benzenes of the formula

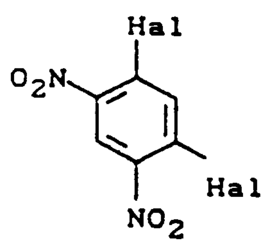

,

in which

Hal represents chlorine or bromine,

are reacted with 2 to 5 moles, preferably 2 to 3 moles, of an arylmethanol of the formula

$Ar-C(R^1,R^2)OH$

in which

$R^1$, $R^2$ and Ar have the said meaning,

and with 2 to 5 equivalents, preferably with 2 to 3 equivalents, of a strong base in the temperature range from 0 to 100 °C, preferably 40 to 70 °C, in the presence of an inert solvent.

**5.** Process according to Claim 4, characterized in that the strong base used is one or more from the group comprising the alkali metals, alkaline earth metals, alkali metal hydroxides, alkaline earth metal

hydroxides, alkali metal carbonates and alkaline earth metal carbonates, preferably from the group alkali metals, alkali metal hydroxides and alkali metal carbonates, and particularly preferentially from the group sodium metal, sodium hydroxide and sodium carbonate.

**6.** Process according to Claim 4, characterized in that the inert solvent used is an aliphatic, aromatic or alkylaromatic hydrocarbon, a halogenated aromatic hydrocarbon or additional arylmethanol, provided this is liquid at the reaction temperature, in an amount of 3 to 20 parts by weight, preferably 3 to 10 parts by weight, relative to the weight of the dihalogeno-dinitro-benzene.

**7.** Process according to Claim 5, characterized in that benzyl alcohol or α-phenyl-ethanol is used as reactant and as solvent in an amount of 3 to 20 parts by weight, preferably 3 to 10 parts by weight, relative to 1 part by weight of dihalogeno-dinitro-benzene.

**8.** Process for the preparation of 4,6-diaminoresorcinol, characterized in that

a) a 1,3-dihalogeno-4,6-dinitro-benzene of the formula

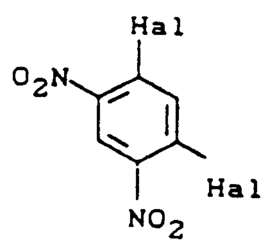

,

in which

Hal represents chlorine or bromine,

is reacted in the liquid phase with 2 to 5 moles, preferably with to 2 to 3 moles, of an arylmethanol of the formula

Ar-C($R^1$,$R^2$)OH,

in which

$R^1$ and $R^2$ independently of one another denote hydrogen or $C_1$-$C_4$-alkyl and

Ar represents $C_6$-$C_{12}$-aryl,

and 2 to 5 equivalents, preferably 2 to 3 equivalents of a strong base in the temperature range from 0 to 100 °C, preferably 40 to 70 °C, and

b) the 1,3-di-arylmethoxy-4,6-dinitro-benzene of the formula

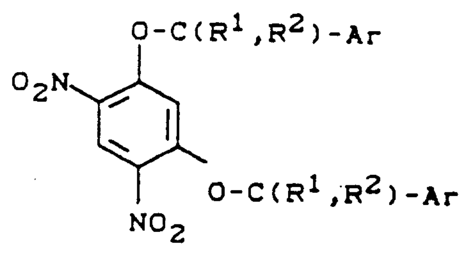

,

in which

$R^1$, $R^2$ and Ar have the said meaning,

obtained in accordance with a), is hydrogenated in heterogeneous phase, using a platinum metal supported catalyst in an amount of 0.1 to 10% by weight, preferably 0.2 to 2% by weight, relative to the weight of the 1,3-di-arylmethoxy-4,6-dinitro-benzene at 1 to 30 bar $H_2$ pressure and in the temperature range from 20 to 100 °C, preferably 20 to 50 °C.

9. Process according to Claim 8, characterized in that the liquid reaction medium used for stage b) is an alcohol, an ether, an aromatic or alkylaromatic hydrocarbon or a mixture of several of these, in an amount of 5 to 25 parts by weight, preferably 7 to 15 parts by weight, relative to 1 part by weight of di-arylmethoxy-dinitro-benzene.

10. Process according to Claim 9, characterized in that the liquid reaction medium used for stage b) is methanol, ethanol, propanol, butanol, tetrahydrofuran, dioxane, toluene, dimethoxy-ethane, diethoxy-ethane, 1,2-dimethoxy-propane and other glycol mono-and di-ethers, acetic acid, propionic acid or a mixture of several of these, and in the case of water-soluble reaction media up to 75% by weight of this reaction medium can be replaced by water or dilute aqueous mineral acid.

## Revendications

1. 1,3-Diarylméthoxy-4,6-dinitrobenzènes de la formule

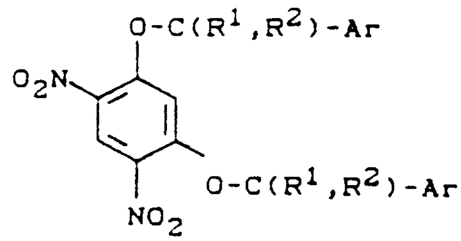

dans laquelle
$R^1$ et $R^2$ indépendamment l'un de l'autre représentent un hydrogène ou un radical alkyle en $C_1$ -$C_4$ et Ar représente un radical aryle en $C_6$ -$C_{12}$.

2. 1,3-Diphénylméthoxy-4,6-dinitrobenzènes de la formule

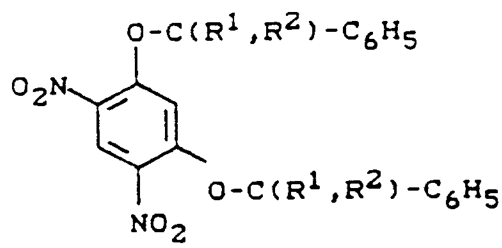

dans laquelle $R^1$ et $R^2$ indépendamment l'un de l'autre représentent un hydrogène ou un radical alkyle en $C_1$ -$C_4$.

3. 1,3-Dibenzyloxy-4,6-dinitrobenzène.

4. Procédé de fabrication des 1,3-diarylméthoxy-4,6-dinitrobenzènes de la formule

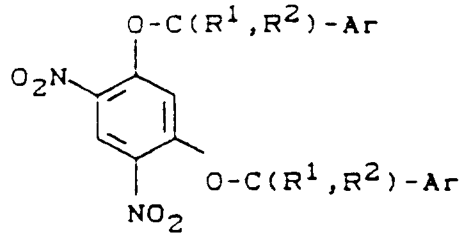

dans laquelle
$R^1$ et $R^2$ indépendamment l'un de l'autre représentent un hydrogène ou un radical alkyle en $C_1$ -$C_4$ et Ar représente un radical aryle en $C_6$ -$C_{12}$,
caractérisé en ce qu'un 1,3-dihalogéno-4,6-dinitrobenzène de la formule

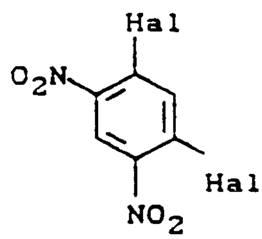

dans laquelle
Hal représente un chlore ou un brome,
est mis à réagir avec 2 à 5 moles, de préférence 2 à 3 moles, d'un arylméthanol de la formule

Ar - C($R^1$, $R^2$) OH

dans laquelle
$R^1$, $R^2$ et Ar ont la signification mentionnée,
et avec 2 à 5 équivalents, de préférence 2 à 3 équivalents, d'une base forte dans un intervalle de température de 0 à 100° C, de préférence de 40 à 70° C, en présence d'un solvant inerte.

5. Procédé selon la revendication 4, caractérisé en ce que la base forte est une ou plusieurs du groupe constitué par les métaux alcalins, les métaux alcalino-terreux, les hydroxydes des métaux alcalins, les hydroxydes des métaux alcalino-terreux, les carbonates des métaux alcalins et les carbonates des métaux alcalino-terreux, de préférence du groupe constitué par les métaux alcalins, les hydroxydes des métaux alcalins et les carbonates des métaux alcalins, de préférence en particulier du groupe constitué par le sodium, l'hydroxyde de sodium et le carbonate de sodium.

6. Procédé selon la revendication 4, caractérisé en ce que le solvant inerte est un hydrocarbure aliphatique, aromatique ou alkylaromatique, un hydrocarbure aromatique halogéné ou un arylméthanol supplémentaire, si celui-ci est liquide à la température de la réaction, dans une quantité de 3 à 20 parties en poids, de préférence 3 à 10 parties en poids, sur base du poids des dihalogénodinitrobenzènes.

7. Procédé selon la revendication 5, caractérisé en ce que l'alcool benzylique ou l'α-phényléthanol est pris comme partenaire de la réaction et comme solvant dans une quantité de 3 à 20 parties en poids, de préférence de 3 à 10 parties en poids, sur base de 1 partie en poids du dihalogénodinitrobenzène.

8. Procédé de fabrication de 4,6-diamino-résorcine, caractérisé en ce que

a) un 1,3-dihalogéno-4,6-dinitrobenzène de la formule

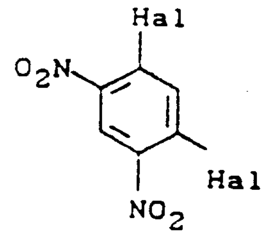

dans laquelle
Hal représente un chlore ou un brome,
est mis en réaction avec 2 à 5 moles, de préférence avec 2 à 3 moles, d'un arylméthanol de la formule

Ar - C($R^1$, $R^2$)OH

dans laquelle
$R^1$, $R^2$ indépendamment l'un de l'autre représentent un hydrogène ou un radical alkyle en $C_1$ -$C_4$ et
Ar représente un radical aryle en $C_6$ -$C_{12}$
et 2 à 5 équivalents, de préférence 2 à 3 équivalents d'une base forte dans l'intervalle de température de 0 à 100° C, de préférence de 40 à 70° C, sont transformés en phase liquide et
b) que le 1,3-diarylméthoxy-4,6-dinitrobenzène, obtenu selon l'étape a), de la formule

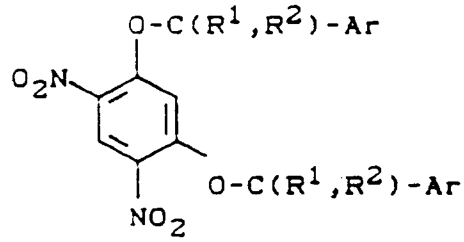

dans laquelle
$R^1$, $R^2$ et Ar ont la signification mentionnée,
est hydrogéné dans une phase hétérogène avec l'utilisation d'un catalyseur de métal du groupe du platine sur support dans une quantité de 0,1 à 10 % en poids, de préférence de 0,2 à 2 % en poids, sur base du poids du 1,3-diarylméthoxy-4,6-dinitrobenzène, sous 1 à 30 bars de pression d'hydrogène et dans un intervalle de température de 20 à 100° C, de préférence de 20 à 50° C.

**9.** Procédé selon la revendication 8, caractérisé en ce que le milieu de réaction liquide pour l'étape b) est un alcool, un éther, un hydrocarbure aromatique ou alkylaromatique ou un mélange de plusieurs d'entre eux mis en oeuvre dans une quantité de 5 à 25 parties en poids, de préférence de 7 à 15 parties en poids, sur base de 1 partie en poids de diarylméthoxydinitrobenzène.

**10.** Procédé selon la revendication 9, caractérisé en ce que le milieu de réaction liquide pour l'étape b) est le méthanol, l'éthanol, le propanol, le butanol, le tétrahydrofuranne, le dioxane, le toluène, le diméthoxyéthane, le diéthoxyéthane, le 1,2-diméthoxypropane et d'autres mono ou diéthers de glycol, l'acide acétique, l'acide propionique ou un mélange de plusieurs d'entre eux, et au cas où le milieu de réaction est soluble dans l'eau, il peut être remplacé jusqu'à 75% en poids de ce milieu de réaction par de l'eau ou un acide minéral aqueux dilué.